# FASCICULE DE BREVET EUROPEEN

(11) **EP 4 630 805 B1**
(45) Date de publication et mention de la délivrance du brevet: **08.07.2026**
(21) Numéro de dépôt: 22846948.2
(22) Date de dépôt: 23.12.2022
(51) Int. Cl.: G01N 33/36, G01N 5/04

(54) **PROCÉDÉ D'ÉVALUATION D'UNE CAPACITÉ DE DÉSORPTION D'UNE LINGETTE D'ESSUYAGE**
VERFAHREN ZUR BEWERTUNG DER DESORPTIONSFÄHIGKEIT EINES WISCHTUCHS
METHOD FOR EVALUATING DESORPTION CAPACITY OF WIPING CLOTH

(30) Priorité: 09.12.2022 FR 2213103
(43) Date de publication de la demande: 15.10.2025
(73) Titulaire: Conformat, SAS, 92500 Rueil-Malmaison (FR)
(72) Inventeur: BAUDOUX, Marie-Hélène, 92310 Sèvres (FR)
(74) Mandataire: Zaboliene, Reda
(86) Numéro de dépôt international: PCT/IB2022/062724
(87) Numéro de publication internationale: WO 2024/121615

(56) Documents cités:
- CN-A- 112 268 829
- MØRETRØ TROND ET AL: "Kitchen cloths: Consumer practices, drying properties and bacterial growth and survival", FOOD CONTROL, BUTTERWORTH, LONDON, GB, vol. 142, 18 June 2022 (2022-06-18), XP087157975, ISSN: 0956-7135, [retrieved on 20220618], DOI: 10.1016/J.FOODCONT.2022.109195
- LI WENBIN ET AL: "A novel method to analyze the moisture liberation of textile fabrics", vol. 9, no. 3, 1 June 2008 (2008-06-01), Seoul, pages 312 - 316, XP093053661, ISSN: 1229-9197, Retrieved from the Internet <URL:https://link.springer.com/content/pdf/10.1007/s12221-008-0050-0.pdf> DOI: 10.1007/s12221-008-0050-0
- CHEN QING ET AL: "Evaluation of water absorption and transport properties of weft knitted polyester fabrics by spontaneous uptake water transport tester and conventional test methods", FIBERS AND POLYMERS, THE KOREAN FIBER SOCIETY, SEOUL, vol. 17, no. 8, 27 August 2016 (2016-08-27), pages 1287 - 1295, XP036043581, ISSN: 1229-9197, [retrieved on 20160827], DOI: 10.1007/S12221-016-6454-3

## Description

Le contexte technique de la présente invention est celui du nettoyage et de la désinfection de salles blanches. Plus particulièrement, l'invention a trait à un procédé d'évaluation des capacités de désorption de textiles d'essuyage à être utilisés dans des environnements de type salles blanches ou salles propres. Un procédé de mesure de la capacité de rétention d'eau d'un tissu est décrit dans le document CN 112 268 829 A, dans lequel un échantillon textile est humidifié dans de l'eau, essoré dans des conditions standardisées, puis pesé afin de déterminer la quantité d'eau retenue par unité de surface ou de masse du tissu.

Dans l'état de la technique, et plus particulièrement dans le domaine du nettoyage dans des environnements sensibles tels que par exemple des salles blanches, on connaît l'utilisation de textiles d'essuyage pour le nettoyage et/ou le rinçage et/ou la désinfection de surfaces, notamment de sols ou de plans de travail par exemple. De tels textiles d'essuyage sont imprégnées d'une solution nettoyante et/ou désinfectante afin de saturer lesdits textiles afin de les rendre adaptés à nettoyer et/ou désinfecter des dispositifs et des surfaces présents dans ces environnements sensibles.

En effet, le domaine particulier des salles blanches conduit à des exigences en termes de nettoyage et/ou de rinçage et/ou de désinfection de l'ordre du micromètre. Il en résulte que les textiles utilisés dans le domaine du nettoyage et/ou du rinçage domestique ne sont pas adaptés à une telle utilisation en salle blanche.

En outre, l'efficacité des textiles d'essuyage n'est pas invariante au cours du temps : plus un textile d'essuyage est utilisé, plus son efficacité est susceptible de diminuer. Par efficacité, on comprend une capacité du textile d'essuyage à désorber la solution de nettoyage et/ou de rinçage et/ou de désinfection sur la surface à nettoyer sur laquelle ledit textile d'essuyage est appliqué, et/ou une capacité du textile d'essuyage à absorber des corps et/ou des liquides présents sur la surface à nettoyer.

Un problème connu réside alors dans l'incapacité à garantir un nettoyage et/ou un rinçage et/ou une désinfection réelle des surfaces à nettoyer et/ou désinfecter, résultant de la perte d'efficacité supposée mais non déterminée des textiles de nettoyage utilisés.

La présente invention a pour objet de proposer un nouveau procédé d'évaluation d'une capacité de désorption d'un textile d'essuyage afin de répondre au moins en grande partie aux problèmes précédents et de conduire en outre à d'autres avantages.

Un autre but de l'invention est de mieux comprendre le comportement des textiles d'essuyage durant leur utilisation.

Un autre but de l'invention est de déterminer des conditions d'utilisation optimale d'un textile d'essuyage dans un environnement de salles blanches ou de salles propres.

Un autre but de l'invention est d'améliorer la qualité des textiles d'essuyage et du nettoyage et/ou de la désinfection réalisée.

Selon un premier aspect de l'invention, on atteint au moins l'un des objectifs précités avec un procédé d'évaluation d'une capacité de désorption d'un textile d'essuyage, le procédé comportant les étapes suivantes :
- une première étape de pesage du textile d'essuyage ;
- une étape d'humidification du textile d'essuyage, l'étape d'humidification comportant une étape de mise en contact du textile d'essuyage avec une solution de nettoyage et/ou de rinçage et/ou de désinfection ;
- une étape d'égouttage du textile d'essuyage humidifié pendant une durée d'égouttage de référence ;
- une deuxième étape de pesage du textile d'essuyage égoutté ;
- une étape d'application du textile d'essuyage égoutté sur une surface à nettoyer et selon une force d'appui prédéterminée du textile d'essuyage égoutté contre la surface à nettoyer ;
- une étape de déplacement du textile d'essuyage égoutté contre la surface à nettoyer et sur une distance prédéterminée, la force d'appui étant maintenue sur la distance prédéterminée ;
- une troisième étape de pesage du textile d'essuyage à l'issue de l'étape de déplacement ;
- une étape de détermination, à partir au moins de la première étape de pesage, de la deuxième étape de pesage et de la troisième étape de pesage d'un volume de solution de nettoyage et/ou de rinçage et/ou de désinfection désorbée par le textile d'essuyage.

Dans le contexte de la présente invention, le textile d'essuyage utilisé dans le procédé conforme au premier aspect de l'invention n'est pas du type de celle utilisée dans le domaine du nettoyage domestique. Il s'agit de textiles d'essuyage ultra propres et, lorsque nécessaire, stériles stérilisées afin de réduire au maximum les risques de contamination particulaires, chimiques et microbiologiques de particules et/ou de bactéries dans les salles blanches lors de la mise en œuvre du procédé de conditionnement conforme au premier aspect de l'invention. De tels textiles d'essuyage sont par exemple conditionnés dans des sachets fermés. La solution de nettoyage et/ou de rinçage et/ou de désinfection est versée dans le sachet afin de pouvoir imprégner les textiles d'essuyage avec une quantité prédéterminée de solution. Un volume de solution de nettoyage et/ou de rinçage et/ou de désinfection introduit dans le sachet est variable selon la nature des textiles d'essuyage , leur capacité d'absorption, leur nombre par sachet ainsi que leur utilisation future : dépoussiérage, nettoyage, désinfection ou rinçage.

D'une manière générale, dans le contexte de la présente invention, le textile d'essuyage peut être une lingette simple couche ou une lingette multicouches. Par ailleurs le textile d'essuyage peut être à usage manuel ou à l'aide d'un accessoire de nettoyage tel que par exemple un balai d'essuyage.

Dans le contexte de la présente invention, les étapes de pesage consiste en la détermination d'une masse du textile d'essuyage mis en œuvre dans le procédé selon l'invention. La détermination de la masse peut être réalisée par tout moyen, et notamment à l'aide d'une balance de précision.

Dans le contexte de la présente invention, l'étape d'égouttage consiste en une évacuation d'un excédent de solution de nettoyage et/ou de rinçage et/ou de désinfection du textile d'essuyage. L'étape d'égouttage peut prendre la forme d'une étape de mise en suspension du textile d'essuyage afin que l'excédent de solution de nettoyage et/ou de rinçage et/ou de désinfection s'écoule hors du textile d'essuyage par simple effet gravitaire. Alternativement, l'étape d'égouttage peut prendre la forme d'une étape d'essorage du textile d'essuyage afin que l'excédent de solution de nettoyage et/ou de rinçage et/ou de désinfection s'écoule hors du textile d'essuyage suite à l'application d'un effort prédéterminé de torsion ou de compression sur ledit textile d'essuyage.

Dans le contexte de la présente invention, l'étape d'application du textile d'essuyage sur la surface à nettoyer comporte une étape de mise en contact du textile d'essuyage sur la surface à nettoyer, . Par suite, le textile d'essuyage peut être maintenu contre la surface à nettoyer sous l'effet de son simple poids ou par l'intermédiaire d'une force d'appui exercée directement ou indirectement sur le textile d'essuyage et en direction de la surface à nettoyer. Ainsi, l'application de la force d'appui sur le textile d'essuyage permet d'augmenter la pression du textile d'essuyage contre la surface à nettoyer.

Dans le contexte de la présente invention, la surface à nettoyer est du type d'une surface plane ou non, horizontale, verticale ou inclinée. La surface à nettoyer peut être du type d'une surface basse, telle que par exemple un sol, ou une surface haute, telle que par exemple un plan de travail, un bureau, une table, une paillasse, une étagère, une cloison, un mur, une porte, objets et matériels utilisés en salles propres. Complémentairement, la surface à nettoyer peut être de n'importe quelle matière : plastique, et notamment PVC, carrelage, inox, verre...

Dans le contexte de la présente invention, l'étape de déplacement consiste en un déplacement du textile d'essuyage contre la surface à nettoyer, la force d'appui définie lors de l'étape d'application étant maintenue durant toute la durée de l'étape de déplacement. De manière préférentielle, le déplacement réalisé durant l'étape de trainé est du type d'un déplacement rectiligne, en ligne droite. En outre, une vitesse de déplacement est préférentiellement constante afin de maintenir constantes et invariantes des interactions entre le textile d'essuyage la surface à nettoyer durant l'étape de déplacement.

Dans le contexte de la présente invention, l'étape de détermination du volume de solution de nettoyage et/ou de rinçage et/ou de désinfection consiste en une évaluation ou une mesure d'un volume de la solution de nettoyage et/ou de rinçage et/ou de désinfection qui a été relarguée par le textile d'essuyage sur la surface à nettoyer durant tout ou partie de l'étape de déplacement. De manière astucieuse, l'étape de détermination comporte une étape de comparaison des masses du textile d'essuyage obtenues lors des différentes mesures, et notamment en comparant la masse du textile d'essuyage avant l'étape de déplacement et la masse dudit textile d'essuyage après l'étape de déplacement.

Ainsi, il est possible de qualifier plus précisément les performances du textile d'essuyage associé à la solution de nettoyage et/ou de rinçage et/ou de désinfection et à la surface à nettoyer considérée. Consécutivement, il est possible de fournir à un utilisateur futur dudit textile d'essuyage des recommandations plus précises quant aux conditions d'utilisation et à un protocole d'utilisation à mettre en place, garantissant ainsi une efficacité supérieure d'un nettoyage et/ou d'une désinfection à l'aide desdits textile d'essuyage par rapport à ceux connus.

Le procédé conforme au premier aspect de l'invention comprend avantageusement au moins un des perfectionnements ci-dessous, les caractéristiques techniques formant ces perfectionnements pouvant être prises seules ou en combinaison :
- la durée d'égouttage de référence est inférieure ou égale à 5 minutes, préférentiellement égale à 2 minutes. Cette configuration avantageuse permet d'évacuer hors du textile d'essuyage un excédent de solution de nettoyage et/ou de rinçage et/ou de désinfection qui nuirait à l'application du textile d'essuyage sur la surface à nettoyer, et son efficacité. En outre, la définition d'une durée d'égouttage de référence permet de rendre reproductible et répétable le procédé conforme au premier aspect de l'invention ;
- la force d'appui est obtenue par l'application d'une masse de référence sur le textile d'essuyage. Cette configuration avantageuse permet de garantir une force d'appui constante et invariante ;
- dans le cas d'un textile d'essuyage utilisé pour le nettoyage d'une surface haute, la masse de référence est supérieure à 2 kg, préférentiellement égale à 2,4 kg. Par surface haute, on comprend que la surface à nettoyer est du type d'une surface d'une table ou d'une paillasse, ou une tablette de rangement, ou une étagère, ou encore une surface d'un mur. D'une manière générale, la surface haute est n'importe quelle surface située au-dessus du niveau du sol et qui n'est pas une surface de marche ;
- a contrario, dans le cas d'un textile d'essuyage utilisé pour le nettoyage d'une surface basse, la masse de référence est supérieure à 1,5 kg, préférentiellement égale à 2 kg. Par surface basse, on comprend que la surface à nettoyer est un sol sur lequel il est possible de marcher ;
- la distance prédéterminée est supérieure ou égale à 2 m linéaire. Cette configuration avantageuse permet de tester le textile d'essuyage sur une distance suffisante pour en mesurer une désorption de la solution de nettoyage et/ou de rinçage et/ou de désinfection ;
- dans le cas d'un textile d'essuyage utilisé pour le nettoyage d'une surface haute, la distance prédéterminée est supérieure à 5 m linéaire, préférentiellement égale à 8 m linéaire. En revanche, dans le cas d'un textile d'essuyage utilisé pour le nettoyage d'une surface basse, la distance prédéterminée est supérieure à 10 m linéaire, préférentiellement égale à 40 m linéaire ;
- dans le cas d'un textile d'essuyage utilisé pour le nettoyage d'une surface basse, le procédé comporte une étape de mise en place du textile d'essuyage sur un balai, l'étape d'application et l'étape de déplacement étant réalisées par l'intermédiaire du balai. L'étape de mise en place du textile d'essuyage est consécutive de l'étape d'égouttage. Cette configuration avantageuse permet ainsi d'autoriser une meilleure application du textile d'essuyage contre la surface à nettoyer, avec application constante de la force d'appui durant l'étape de déplacement ;
- l'étape de mise en contact comporte une étape d'immersion du textile d'essuyage dans la solution de nettoyage et/ou de rinçage et/ou de désinfection. Cette configuration permet ainsi d'imprégner le textile d'essuyage dans la solution de nettoyage et/ou de rinçage et/ou de désinfection. A cet effet, le textile d'essuyage est placé dans un contenant autorisant à la fois le dépôt d'une ou plusieurs textiles d'essuyage , et le remplissage, partiel ou total, du contenant avec la solution de nettoyage et/ou de rinçage et/ou de désinfection. Le contenant peut être du type d'un sachet, de préférence souple, avantageusement autoporté, refermable ou non. Alternativement, le contenant peut être du type d'un contenant solide, tel que par exemple une bassine. Un volume de solution nettoyante et/ou désinfectante versé dans le contenant est déterminé en fonction d'un nombre de textile d'essuyage et/ou un type de textile d'essuyage et/ou un volume du contenant et/ou un type de solution de nettoyage et/ou de rinçage et/ou de désinfection et/ou un type de surface à nettoyer ;
- l'étape d'immersion est réalisée pendant une durée d'immersion de référence, par exemple inférieure ou égale à une minute. Cette configuration avantageuse permet de laisser un temps suffisant au textile d'essuyage pour s'imprégner de la solution de nettoyage et/ou de rinçage et/ou de désinfection ;
- le procédé comporte une pluralité d'étapes de pesages intermédiaires réalisées durant l'étape de déplacement du textile d'essuyage, l'étape de détermination du volume de solution de nettoyage et/ou de rinçage et/ou de désinfection désorbée par le textile d'essuyage étant réalisée à partir de la pluralité d'étapes de pesages intermédiaires. Cette configuration avantageuse permet ainsi de mesurer une désorption progressive de la solution de nettoyage et/ou de rinçage et/ou de désinfection sur la surface de nettoyage par le textile d'essuyage
- les étapes de pesages intermédiaires sont réalisées à intervalles constants durant l'étape de déplacement, une distance entre deux pesages intermédiaires successifs étant constante. Ainsi, par exemple, dans le cas d'un textile d'essuyage utilisé pour le nettoyage d'une surface haute, une longueur des intervalles est inférieure à 2 m, préférentiellement égale à 1 m. A contrario, dans le cas d'un textile d'essuyage utilisé pour le nettoyage d'une surface basse, une longueur des intervalles est par exemple supérieure à 2 m, préférentiellement égale à 2,5 m ;
- l'étape de détermination du volume de solution de nettoyage et/ou de rinçage et/ou de désinfection désorbée par le textile d'essuyage comporte une étape de détermination du volume désorbé pour chaque intervalle. Cette configuration avantageuse permet ainsi de mesurer une désorption progressive de la solution de nettoyage et/ou de rinçage et/ou de désinfection sur la surface de nettoyage par le textile d'essuyage et d'évaluer à partir de quel intervalle ou distance le volume de solution de nettoyage et/ou de rinçage et/ou de désinfection laissée sur la surface à nettoyer n'est plus suffisant pour assurer le nettoyage et/ou le rinçage et/ou la désinfection. Ainsi, il est possible de déterminer l'intervalle ou la distance à partir duquel l'opération de nettoyage et/ou de rinçage et/ou de désinfection n'est plus considérée comme efficace, l'essuyage pouvant alors être arrêté. Ainsi, cette configuration avantageuse permet de réaliser un étalonnage du textile d'essuyage et de la solution de nettoyage et/ou de rinçage et/ou de désinfection afin de déterminer précisément des conditions optimales d'utilisation.

Des modes de réalisation variés de l'invention sont prévus, intégrant selon l'ensemble de leurs combinaisons possibles les différentes caractéristiques optionnelles exposées ici.

D'autres caractéristiques et avantages de l'invention apparaîtront encore au travers de la description qui suit d'une part, et de plusieurs exemples de réalisation donnés à titre indicatif et non limitatif en référence aux dessins schématiques annexés d'autre part, sur lesquels :

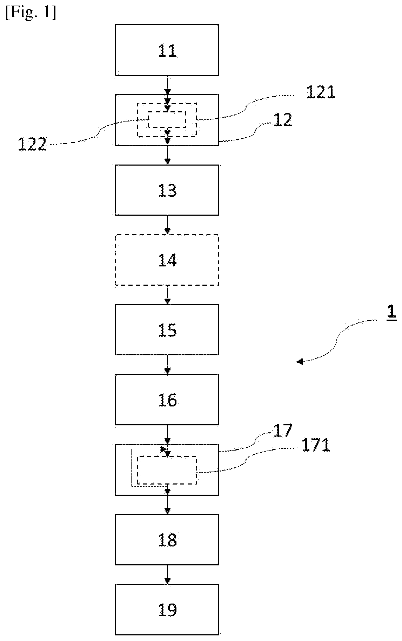

[Fig.1] illustre une vue synoptique du procédé d'évaluation d'une capacité de désorption d'un textile d'essuyage conforme au premier aspect de l'invention.

Bien entendu, les caractéristiques, les variantes et les différentes formes de réalisation de l'invention peuvent être associées les unes avec les autres, selon diverses combinaisons, dans la mesure où elles ne sont pas incompatibles ou exclusives les unes des autres. On pourra notamment imaginer des variantes de l'invention ne comprenant qu'une sélection de caractéristiques décrites par la suite de manière isolée des autres caractéristiques décrites, si cette sélection de caractéristiques est suffisante pour conférer un avantage technique ou pour différencier l'invention par rapport à l'état de la technique antérieure.

En particulier toutes les variantes et tous les modes de réalisation décrits sont combinables entre eux si rien ne s'oppose à cette combinaison sur le plan technique.

Sur les figures, les éléments communs à plusieurs figures conservent la même référence.

En référence à la FIGURE 1, l'invention adresse un procédé 1 d'évaluation d'une capacité de désorption d'un textile d'essuyage du type de ceux utilisés dans des environnements contrôlés tels que des salles blanches ou des salles propres. Le procédé 1 selon l'invention comporte les étapes suivantes :
- une première étape de pesage 11 du textile d'essuyage ;
- une étape d'humidification 12 du textile d'essuyage, l'étape d'humidification 12 comportant une étape de mise en contact 121 du textile d'essuyage avec une solution de nettoyage et/ou de rinçage et/ou de désinfection ;
- une étape d'égouttage 13 du textile d'essuyage humidifié pendant une durée d'égouttage de référence ;
- une deuxième étape de pesage 15 du textile d'essuyage égoutté ;
- une étape d'application 16 de la lingette de nettoyage égouttée sur une surface à nettoyer et selon une force d'appui prédéterminée du textile d'essuyage égoutté contre la surface à nettoyer ;
- une étape de déplacement du textile d'essuyage égoutté contre la surface à nettoyer et sur une distance prédéterminée, la force d'appui étant maintenue sur la distance prédéterminée ;
- une troisième étape de pesage 18 du textile d'essuyage à l'issue de l'étape de déplacement ;
- une étape de détermination 19, à partir au moins de la première étape de pesage 11, de la deuxième étape de pesage 15 et de la troisième étape de pesage 18 d'un volume de solution de nettoyage et/ou de rinçage et/ou de désinfection désorbée par le textile d'essuyage.

La première étape de pesage 11 du textile d'essuyage est réalisée avant mise en contact avec la solution de nettoyage et/ou de rinçage et/ou de désinfection afin de déterminer une masse sèche dudit textile d'essuyage.

La deuxième étape de pesage 15 du textile d'essuyage est réalisée juste après l'étape d'égouttage 13 et permet de mesurer la masse du textile d'essuyage imprégnée de la solution de nettoyage et/ou de rinçage et/ou de désinfection

La troisième étape de pesage 18 du textile d'essuyage est réalisée après l'étape de déplacement dudit textile d'essuyage sur la surface à nettoyer et permet ainsi de mesurer une quantité restante de la solution de nettoyage et/ou de rinçage et/ou de désinfection encore imprégnée sur le textile d'essuyage, permettant donc de déterminer, par déduction avec les première et deuxième mesure réalisées précédemment, une quantité de la solution de nettoyage et/ou de rinçage et/ou de désinfection désorbée par le textile d'essuyage sur la surface à nettoyer durant l'étape de déplacement.

De manière avantageuse, le procédé 1 conforme à l'invention peut être réalisé selon un nombre d'itération supérieur à 1, par exemple égal à 3, afin d'évaluer la répétabilité des mesures ainsi réalisées et de déterminer une valeur moyenne de la quantité de solution de nettoyage et/ou de rinçage et/ou de désinfection désorbée par le textile d'essuyage durant l'étape de déplacement.

De manière avantageuse, le textile d'essuyage utilisé dans la procédé 1 selon l'invention présente une surface de plusieurs centaines de centimètres-carrés afin de permettre une application efficace du textile d'essuyage sur la surface à nettoyer.

Durant l'étape d'application 16 et l'étape de déplacement, la force d'appui est obtenue par l'application d'une masse de référence sur le textile d'essuyage afin de garantir une force d'appui constante et invariante durant l'exécution du procédé 1 selon l'invention.

Par ailleurs, l'étape de mise en contact 121 comporte une étape d'immersion 122 du textile d'essuyage dans la solution de nettoyage et/ou de rinçage et/ou de désinfection afin d'imprégner la lingette nettoyante dans la solution de nettoyage et/ou de rinçage et/ou de désinfection. A cet effet, le textile d'essuyage est placé dans un contenant autorisant à la fois le dépôt d'une ou plusieurs textiles d'essuyage, et le remplissage, partiel ou total, du contenant avec la solution de nettoyage et/ou de rinçage et/ou de désinfection. Un volume de solution nettoyante et/ou désinfectante versé dans le contenant est déterminé en fonction d'un nombre de textile d'essuyage et/ou un type de textile d'essuyage et/ou un volume du contenant et/ou un type de solution de nettoyage et/ou de rinçage et/ou de désinfection et/ou un type de surface à nettoyer.

Par suite, l'étape d'immersion 122 est réalisée pendant une durée d'immersion de référence, préférentiellement égale à une minute afin de laisser le temps au textile d'essuyage d'absorber et de s'imprégner de la solution de nettoyage et/ou de rinçage et/ou de désinfection.

Le procédé 1 selon l'invention présente deux variantes de réalisation en fonction de la destination des textiles d'essuyage testés.

Dans le cas où le textile d'essuyage est destiné à être utilisée sur une surface basse, telle qu'un sol de la salle propre ou de la salle blanche par exemple, alors :
- la masse de référence est supérieure à 1,5 kg, préférentiellement égale à 2 kg ; et/ou
- la distance prédéterminée est supérieure à 10 m linéaire, préférentiellement égale à 40 m linéaire ; et/ou
- le procédé 1 selon l'invention comporte une étape de mise en place 14 du textile d'essuyage sur un balai, l'étape d'application 16 et l'étape de déplacement étant réalisées par l'intermédiaire du balai. L'étape de mise en place 14 du textile d'essuyage est consécutive de l'étape d'égouttage 13.

Dans le cas où le textile d'essuyage est destiné à être utilisée sur une surface haute, telle qu'une surface de travail d'un bureau, d'une paillasse, un mur ou une porte par exemple, alors :
- la masse de référence est supérieure à 2 kg, préférentiellement égale à 2,4 kg ; et/ou
- la distance prédéterminée est supérieure à 5 m linéaire, préférentiellement égale à 8 m linéaire.

Dans l'une ou l'autre des variantes de réalisation évoquées ici, il peut être avantageux de mesurer la résorption de la solution de nettoyage et/ou de rinçage et/ou de désinfection hors du textile d'essuyage à plusieurs reprises et au fur et à mesure de l'avancement du procédé 1 selon l'invention, afin de pouvoir mesurer une évolution temporelle des capacités du textile d'essuyage testé, en regard de la surface à nettoyer d'une part et de la solution de nettoyage et/ou de rinçage et/ou de désinfection d'autre part.

A cet effet, le procédé 1 comporte avantageusement une ou plusieurs étapes de pesages 11, 15, 171, 18 intermédiaires 171 réalisées durant l'étape de déplacement du textile d'essuyage, l'étape de détermination 19 du volume de solution de nettoyage et/ou de rinçage et/ou de désinfection désorbée par le textile d'essuyage étant réalisée à partir de la pluralité d'étapes de pesages 11, 15, 171, 18 intermédiaires 171. Chaque étape de pesage intermédiaire 171 permet ainsi de déterminer une quantité de solution de nettoyage et/ou de rinçage et/ou de désinfection désorbée sur la surface de nettoyage par le textile d'essuyage. De manière avantageuse, les étapes de pesages 11, 15, 171, 18 intermédiaires 171 sont réalisées à intervalles constants durant l'étape de déplacement, une distance entre deux pesages intermédiaires 171 successifs étant constante. Ainsi, par exemple, dans le cas d'un textile d'essuyage utilisé pour le nettoyage d'une surface haute, une longueur des intervalles est inférieure à 2 m, préférentiellement égale à 1 m. A contrario, dans le cas d'un textile d'essuyage utilisé pour le nettoyage d'une surface basse, une longueur des intervalles est par exemple supérieure à 2 m, préférentiellement égale à 2,5 m.

En synthèse, l'invention concerne un procédé 1 d'évaluation d'une capacité de désorption d'un textile d'essuyage, le procédé 1 comportant plusieurs étapes de pesage du textile d'essuyage, réalisées successivement avant et après utilisation du textile d'essuyage, imprégné d'une solution de nettoyage et/ou de rinçage et/ou de désinfection lors d'une étape d'humidification 12, afin de mesurer l'évolution d'une quantité de solution de nettoyage et/ou de rinçage et/ou de désinfection retenue par le textile d'essuyage. Le textile d'essuyage est utilisé sur une surface à nettoyer de test sur laquelle, au cours d'une étape d'application 16, le textile d'essuyage est pressé contre la surface à nettoyer et déplacé sur une distance prédéterminée lors d'une étape de déplacement.

Bien sûr, l'invention n'est pas limitée aux exemples qui viennent d'être décrits et de nombreux aménagements peuvent être apportés à ces exemples sans sortir du cadre de l'invention. Notamment, les différentes caractéristiques, formes, variantes et modes de réalisation de l'invention peuvent être associées les unes avec les autres selon diverses combinaisons dans la mesure où elles ne sont pas incompatibles ou exclusives les unes des autres. En particulier toutes les variantes et modes de réalisation décrits précédemment sont combinables entre eux.

## Revendications

1. Procédé (1) d'évaluation d'une capacité de désorption d'un textile, le procédé (1) comportant les étapes suivantes :
- une première étape de pesage (11) du textile;
- une étape d'humidification (12) du textile ;
- une étape d'égouttage (13) du textile humidifié pendant une durée d'égouttage de référence ;
- une deuxième étape de pesage (15) du textile égoutté ;
- une étape d'application (16) du textile égoutté sur une surface à nettoyer et selon une force d'appui prédéterminée du textile égoutté contre la surface à nettoyer ;
**caractérisé en ce que** le textile est du type d'un textile d'essuyage ;
et **en ce que** l'étape d'humidification (12) comporte une étape de mise en contact (121) du textile d'essuyage avec une solution de nettoyage et/ou de rinçage et/ou de désinfection ;
et **en ce que** le procédé (1) comporte une étape de déplacement du textile d'essuyage égoutté contre la surface à nettoyer et sur une distance prédéterminée, la force d'appui étant maintenue sur la distance prédéterminée, une troisième étape de pesage (18) du textile d'essuyage à l'issue de l'étape de déplacement, et une étape de détermination (19), à partir au moins de la première étape de pesage (11), de la deuxième étape de pesage (15) et de la troisième étape de pesage (18) d'un volume de solution de nettoyage et/ou de rinçage et/ou de désinfection désorbée par le textile d'essuyage.

2. Procédé (1) selon la revendication précédente, dans lequel la force d'appui est obtenue par l'application d'une masse de référence sur le textile d'essuyage.

3. Procédé (1) selon la revendication 2, dans lequel, dans le cas d'un textile d'essuyage utilisé pour le nettoyage d'une surface haute, la masse de référence est supérieure à 2 kg, préférentiellement égale à 2,4 kg.

4. Procédé (1) selon la revendication 2, dans lequel, dans le cas d'un textile d'essuyage utilisé pour le nettoyage d'une surface basse, la masse de référence est supérieure à 1,5 kg, préférentiellement égale à 2 kg.

5. Procédé (1) selon l'une quelconque des revendications 2 ou 3, dans lequel, dans le cas d'un textile d'essuyage utilisé pour le nettoyage d'une surface haute, la distance prédéterminée est supérieure à 5 m linéaire, préférentiellement égale à 8 m linéaire.

6. Procédé (1) selon l'une quelconque des revendications 2 ou 4, dans lequel, dans le cas d'un textile d'essuyage utilisé pour le nettoyage d'une surface basse, la distance prédéterminée est supérieure à 10 m linéaire, préférentiellement égale à 40 m linéaire.

7. Procédé (1) selon l'une quelconque des revendications précédentes, dans lequel l'étape de mise en contact (121) comporte une étape d'immersion (122) du textile d'essuyage dans la solution de nettoyage et/ou de rinçage et/ou de désinfection.

8. Procédé (1) selon l'une quelconque des revendications précédentes, dans lequel le procédé (1) comporte une pluralité d'étapes de pesages (11, 15, 171, 18) intermédiaires (171) réalisées durant l'étape de déplacement du textile d'essuyage, l'étape de détermination (19) du volume de solution de nettoyage et/ou de rinçage et/ou de désinfection désorbée par le textile d'essuyage étant réalisée à partir de la pluralité d'étapes de pesages (11, 15, 171, 18) intermédiaires (171).

9. Procédé (1) selon la revendication précédente, dans lequel les étapes de pesages (11, 15, 171, 18) intermédiaires (171) sont réalisées à intervalles constants durant l'étape de déplacement, une distance entre deux pesages intermédiaires (171) successifs étant constante.

10. Procédé (1) selon l'une quelconque des revendications 8 ou 9, dans lequel l'étape de détermination (19) du volume de solution de nettoyage et/ou de rinçage et/ou de désinfection désorbée par le textile d'essuyage comporte une étape de détermination (19) du volume désorbé pour chaque intervalle.

## Patentansprüche

1. Ein Verfahren (1) zur Bewertung einer Desorptionsfähigkeit eines Textils, wobei das Verfahren (1) die folgenden Schritte umfasst:
- einen ersten Schritt des Wiegens (11) des Textils;
- einen Schritt der Befeuchtung (12) des Textils;
- einen Schritt des Abtropfens (13) des befeuchteten Textils während einer Referenz-Abtropfdauer;
- einen zweiten Schritt des Wiegens (15) des abgetropften Textils;
- einen Schritt des Aufbringens (16) des abgetropften Textils auf eine zu reinigende Oberfläche und gemäß einer vorbestimmten Andruckkraft des abgetropften Textils gegen die zu reinigende Oberfläche;
**dadurch gekennzeichnet, dass** das Textil von der Art eines Wischtuchs ist;
und dass der Schritt der Befeuchtung (12) einen Schritt des In-Kontakt-Bringens (121) des Wischtuchs mit einer Reinigungs- und/oder Spül- und/oder Desinfektionslösung umfasst;
und dass das Verfahren (1) einen Schritt des Bewegens des abgetropften Wischtuchs gegen die zu reinigende Oberfläche und über eine vorbestimmte Distanz umfasst, wobei die Andruckkraft über die vorbestimmte Distanz aufrechterhalten wird, einen dritten Schritt des Wiegens (18) des Wischtuchs im Anschluss an den Schritt des Bewegens, und einen Schritt des Bestimmens (19), ausgehend zumindest von dem ersten Schritt des Wiegens (11), dem zweiten Schritt des Wiegens (15) und dem dritten Schritt des Wiegens (18), eines Volumens der Reinigungs- und/oder Spül- und/oder Desinfektionslösung, das durch das Wischtuch desorbiert wurde.

2. Das Verfahren (1) nach dem vorhergehenden Anspruch, wobei die Andruckkraft durch das Aufbringen einer Referenzmasse auf das Wischtuch erhalten wird.

3. Das Verfahren (1) nach Anspruch 2, wobei im Fall eines Wischtuchs, das für die Reinigung einer hohen Oberfläche verwendet wird, die Referenzmasse größer als 2 kg ist, vorzugsweise gleich 2.4 kg ist.

4. Das Verfahren (1) nach Anspruch 2, wobei im Fall eines Wischtuchs, das für die Reinigung einer niedrigen Oberfläche verwendet wird, die Referenzmasse größer als 1.5 kg ist, vorzugsweise gleich 2 kg ist.

5. Das Verfahren (1) nach einem der Ansprüche 2 oder 3, wobei im Fall eines Wischtuchs, das für die Reinigung einer hohen Oberfläche verwendet wird, die vorbestimmte Distanz größer als 5 lineare Meter ist, vorzugsweise gleich 8 lineare Meter ist.

6. Das Verfahren (1) nach einem der Ansprüche 2 oder 4, wobei im Fall eines Wischtuchs, das für die Reinigung einer niedrigen Oberfläche verwendet wird, die vorbestimmte Distanz größer als 10 lineare Meter ist, vorzugsweise gleich 40 lineare Meter ist.

7. Das Verfahren (1) nach einem der vorhergehenden Ansprüche, wobei der Schritt des In-Kontakt-Bringens (121) einen Schritt des Eintauchens (122) des Wischtuchs in die Reinigungs- und/oder Spül- und/oder Desinfektionslösung umfasst.

8. Das Verfahren (1) nach einem der vorhergehenden Ansprüche, wobei das Verfahren (1) eine Vielzahl von dazwischenliegenden (171) Schritten des Wiegens (11, 15, 171, 18) umfasst, die während des Schritts des Bewegens des Wischtuchs durchgeführt werden, wobei der Schritt des Bestimmens (19) des Volumens der Reinigungs- und/oder Spül- und/oder Desinfektionslösung, das durch das Wischtuch desorbiert wurde, ausgehend von der Vielzahl von dazwischenliegenden (171) Schritten des Wiegens (11, 15, 171, 18) durchgeführt wird.

9. Das Verfahren (1) nach dem vorhergehenden Anspruch, wobei die dazwischenliegenden (171) Schritte des Wiegens (11, 15, 171, 18) in konstanten Intervallen während des Schritts des Bewegens durchgeführt werden, wobei eine Distanz zwischen zwei aufeinanderfolgenden dazwischenliegenden Wägungen (171) konstant ist.

10. Das Verfahren (1) nach einem der Ansprüche 8 oder 9, wobei der Schritt des Bestimmens (19) des Volumens der Reinigungs- und/oder Spül- und/oder Desinfektionslösung, das durch das Wischtuch desorbiert wurde, einen Schritt des Bestimmens (19) des desorbierten Volumens für jedes Intervall umfasst.

## Claims

1. A method (1) for evaluating a desorption capacity of a textile, the method (1) comprising the following steps:
- a first weighing step (11) of the textile;
- a humidification step (12) of the textile;
- a draining step (13) of the humidified textile for a reference draining duration;
- a second weighing step (15) of the drained textile;
- a step (16) of applying the drained textile onto a surface to be cleaned and according to a predetermined pressing force of the drained textile against the surface to be cleaned;
**characterized in that** the textile is of the wiping cloth type;
and **in that** the humidification step (12) comprises a step (121) of bringing the wiping cloth into contact with a cleaning and/or rinsing and/or disinfecting solution;
and **in that** the method (1) comprises a step of moving the drained wiping cloth against the surface to be cleaned and over a predetermined distance, the pressing force being maintained over the predetermined distance, a third weighing step (18) of the wiping cloth at the end of the movement step, and a determination step (19), based at least on the first weighing step (11), the second weighing step (15), and the third weighing step (18), of a volume of cleaning and/or rinsing and/or disinfecting solution desorbed by the wiping cloth.

2. The method (1) according to the preceding claim, wherein the pressing force is obtained by applying a reference mass to the wiping cloth.

3. The method (1) according to claim 2, wherein, in the case of a wiping cloth used for cleaning a high surface, the reference mass is greater than 2 kg, preferably equal to 2.4 kg.

4. The method (1) according to claim 2, wherein, in the case of a wiping cloth used for cleaning a low surface, the reference mass is greater than 1.5 kg, preferably equal to 2 kg.

5. The method (1) according to any one of claims 2 or 3, wherein, in the case of a wiping cloth used for cleaning a high surface, the predetermined distance is greater than 5 linear m, preferably equal to 8 linear m.

6. The method (1) according to any one of claims 2 or 4, wherein, in the case of a wiping cloth used for cleaning a low surface, the predetermined distance is greater than 10 linear m, preferably equal to 40 linear m.

7. The method (1) according to any one of the preceding claims, wherein the contact step (121) comprises a step (122) of immersing the wiping cloth in the cleaning and/or rinsing and/or disinfecting solution.

8. The method (1) according to any one of the preceding claims, wherein the method (1) comprises a plurality of intermediate (171) weighing steps (11, 15, 171, 18) carried out during the step of moving the wiping cloth, the step of determining (19) the volume of cleaning and/or rinsing and/or disinfecting solution desorbed by the wiping cloth being carried out based on the plurality of intermediate (171) weighing steps (11, 15, 171, 18).

9. The method (1) according to the preceding claim, wherein the intermediate (171) weighing steps (11, 15, 171, 18) are carried out at constant intervals during the movement step, a distance between two successive intermediate weighings (171) being constant.

10. The method (1) according to any one of claims 8 or 9, wherein the step (19) of determining the volume of cleaning and/or rinsing and/or disinfecting solution desorbed by the wiping cloth comprises a step (19) of determining the volume desorbed for each interval.
